# FASCICULE DE BREVET EUROPEEN

(11) **EP 0 946 433 B1**
(45) Date de publication et mention de la délivrance du brevet: **19.04.2000**
(21) Numéro de dépôt: 97911302.4
(22) Date de dépôt: 20.10.1997
(51) Int. Cl.: C02F 3/30, C02F 3/12, C02F 3/00

(54) **PROCEDE D'ELIMINATION DU PHOSPHORE CONTENU DANS DES EFFLUENTS**
VERFAHREN ZUR ENTFERNUNG VON PHOSPHOR AUS ABWASSER
METHOD FOR ELIMINATING PHOSPHORUS CONTAINED IN EFFLUENTS

(30) Priorité: 28.11.1996 FR 9614609
(43) Date de publication de la demande: 06.10.1999
(73) Titulaire: DEGREMONT, 92508 Rueil-Malmaison Cédex (FR)
(72) Inventeur: CHUDOBA, Pavel, F-78230 Le Pecq (FR); PUJOL, Roger, F-78400 Chatou (FR)
(74) Mandataire: Armengaud Ainé, Alain
(86) Numéro de dépôt international: FR9701877
(87) Numéro de publication internationale: WO9823542

(56) Documents cités:
- DE-A- 2 218 180
- DE-A- 3 902 626
- DE-A- 4 133 805

## Description

La présente invention concerne une régulation optimisée des procédés d'élimination du phosphore contenu dans des effluents notamment des eaux usées, simultanément par voie biologique et physico-chimique.

Le principe de la déphosphatation biologique est basé sur la capacité de certaines bactéries, appelées poly-P, d'accumuler le phosphore sous forme instable de complexes polyphosphatés ou de l'adénosine triphosphate (ATP). Cette accumulation a lieu en conditions aérobies ou anoxiques, en présence d'oxygène dissous ou de nitrates. Cette accumulation aérobie est précédée par une étape anaérobie en amont, lors de laquelle, les bactéries poly-P effectuent une fermentation anaérobie sur la matière organique issue de l'eau usée, afin de la transformer en molécules facilement assimilables : les acides gras volatils (AGV). Ensuite, ces acides gras volatils sont transformés par les bactéries poly-P en matières de réserve, appelées poly beta-hydroxy alkanoates (PHA). Le composé le plus souvent lié à ces transformations est le poly beta-hydroxy butyrate (PHB). Pour effectuer cette transformation, les bactéries poly-P puisent de l'énergie à partir de la dépolymérisation des réserves intracellulaires en polyphosphate. Ce processus s'accompagne du relargage du phosphore soluble, sous forme d'orthophosphates, dans le milieu. Le retour en conditions aérobies ou anoxiques est accompagné par le processus inverse. Les bactéries poly-P effectuent une accumulation de phosphore soluble sous forme de polyphosphates intracellulaires (ATP), avec comme source d'énergie, le PHB accumulé précédemment. Ce processus est accompagné d'une disparition du phosphore de la phase soluble.

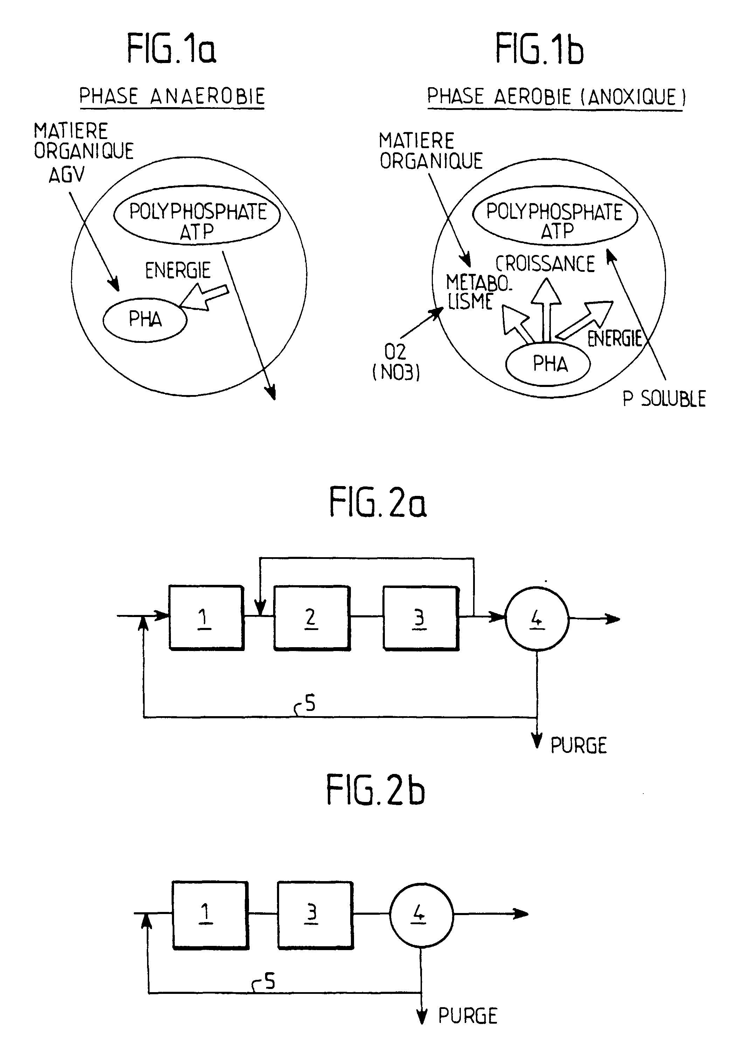

Sur le schéma des figures 1a et 1b des dessins annexés, on a résumé les mécanismes mis en jeu lors des phases anaérobie (figure 1a) et aérobie (anoxique - figure 1b).

Sur les figures 2a et 2b des dessins annexés, on a illustre, a titre d'exemples non limitatifs, deux configurations possibles de la filière mettant en oeuvre la déphosphatation biologique. Ces configurations comportent une séquence des zones anaérobie 1, anoxique 2 et aérobie 3 (figure 2a) ou anaérobie 1 et aérobie 3 (figure 2b). La filière comporte en outre un clarificateur 4 (clarificateur secondaire) dont la fonction est de séparer les boues biologiques de l'effluent traité, étant entendu que cette installation comporte en amont des zones 1, 2 et 3, les moyens classiques de dessablage, dégraissage et éventuellement de clarification primaire. Une partie des boues provenant du clarificateur 4 est recyclée en tête vers la zone anaérobie 1, le reste allant à la purge.

Un exemple d'un procédé de déphosphatation biologique est décrit dans DE-A-3902626. Dans cette publication, des composés organiques solubles (carbone organique) sont ajoutés dans l'eau à traiter et l'on mesure la turbidité de l'eau et la teneur en phosphates en un seul endroit, dans le bassin d'aération, après une séparation des boues, et grâce aux deux mesures ainsi obtenues, on réalise une régulation de l'apport d'air, en phase d'aération ainsi que l'addition d'acide acétique. Ce procédé connu a pour objet d'accélérer la déphosphatation biologique et plus particulièrement la dénitrification. Il est mentionné dans cette publication antérieure que la déphosphatation par voie physico-chimique (addition des réactifs précipitant le phosphore) constitue un inconvénient.

L'expérience montre que, dans la pratique, ce processus est souvent difficile à maîtriser, car les formes du phosphore accumulé à l'intérieur des cellules bactériennes sont très instables. Il suffit d'un temps de séjour relativement court en milieu anaérobie, pour qu'un relargage du phosphore en phase soluble se produise.

Le manque de fiabilité des procédés mettant en oeuvre une déphosphatation biologique est souvent dû à deux facteurs principaux :
- Une indisponibilité ou une insuffisance de la source du carbone facilement assimilable se traduisant par une fraction trop faible et souvent variable dans le temps de la DCO facilement assimilable dans l'eau usée ;
- Un relargage incontrôlé du phosphore en phase liquide pendant les temps de séjour prolongés dans le clarificateur, ce qui se traduit pas des problèmes d'exploitation.

Ces facteurs permettent d'expliquer les défaillances des installations classiques de déphosphatation biologique. Pour assurer un niveau de traitement fiable, on associe en général dans la même filière la déphosphatation biologique et la déphosphatation physico-chimique. On parle alors de déphosphatation combinée.

Une fraction du phosphore de l'eau usée est ainsi éliminée par voie biologique, en présence du carbone organique facilement assimilable de l'eau usée. L'importance de cette fraction du phosphore assimilé par les bactéries poly-P dépend de la concentration en matière facilement assimilable dans l'eau usée. Ainsi, la contribution de la déphosphatation biologique à l'élimination globale du phosphore est très variable, entre 40 et 90%.

Pour assurer le rendement d'élimination du phosphore exigé par le niveau de rejet requis, le reste du phosphore doit être éliminé par la précipitation physico-chimique. Cette précipitation a généralement lieu dans la zone d'aération , suite à l'injection d'un produit précipitant, par exemple des sels ferreux ou ferriques. La quantité du produit injecté est calculée en fonction de la concentration du phosphore à éliminer par précipitation, multipliée par un facteur stoechiométrique.

Jusqu'à présent, la quantité du produit précipitant à injecter était estimée de façon empirique ou bien le produit précipitant était injecté en excès pour obtenir l'élimination complète du phosphore. Parfois, les doses peuvent être asservies à la mesure du phosphore soluble dans l'eau usée à l'entrée de la station.

Ces modes d'injection ne peuvent cependant pas garantir, de façon fiable, le niveau parfois très sévère de rejet en matière phosphorée. Lorsque la précipitation du phosphore est combinée aux mécanismes de la déphosphatation biologique, les fractions du phosphore incorporé par les bactéries poly-P se trouvent toujours en état très instable. Il suffit alors d'un problème au niveau de la clarification secondaire, un défaut d'exploitation ou tout simplement un dysfonctionnement de certains équipements, pour que le relargage du phosphore dans la phase liquide au cours de la clarification ait lieu. Ceci entraîne une détérioration immédiate du niveau de rejet en matière phosphorée et un non-respect des normes de rejet.

Afin de pallier les inconvénients des solutions selon la technique antérieure rappelées ci-dessus, la présente invention apporte un procédé de traitement des eaux usées simultanément par voie biologique et physico-chimique, mettant en oeuvre une déphosphatation biologique, ce procédé comportant des étapes de traitements anaérobie, anoxique et aérobie, suivies d'une étape de clarification secondaire, l'eau usée étant mélangée à la boue biologique recyclée à partir de l'étape de clarification secondaire dans la première étape anaérobie, afin d'effectuer un relargage du phosphore dans la phase soluble par les bactéries poly-P, ledit mélange subissant ensuite les étapes traditionnelles de traitements anoxique et aérobie, la boue activée étant séparée de l'eau traitée dans l'étape de clarification secondaire et elle est ensuite partiellement recyclée dans l'étape anaérobie, en tête de la ligne de traitement, et partiellement extraite du système, ce procédé étant caractérisé en ce que :
- la concentration en phosphore soluble de l'eau traitée est mesurée sur au- moins deux endroits différents de la ligne de traitement : à l'entrée de l'étape de clarification secondaire afin d'obtenir une mesure P1 et à la sortie de la ligne de traitement pour obtenir une mesure P2, la mesure P1 déterminant les doses du produit précipitant le phosphore soluble par voie physico-chimique devant être injectées dans la liqueur d'au moins l'un des bassins ou s'effectuent les étapes anoxique ou aérobie en amont de la clarification secondaire ;
- la mesure P2 est comparée en permanence à la mesure P1 afin de détecter un éventuel relargage du phosphore au niveau de la clarification secondaire et,
- lorsque la différence des deux mesures (ΔP1-P2) est constatée, on déclenche au moins l'une des deux mesures suivantes :
- on réalise une injection supplémentaire du produit précipitant, avant la clarification secondaire, afin que la précipitation du phosphore relargué au cours de l'étape de clarification secondaire puisse avoir lieu grâce à l'excès du produit précipitant injecté;
- on modifie le débit de recirculation des boues de l'étape de clarification vers la première étape anaérobie, afin de réduire leur temps de séjour durant cette étape de clarification.

Selon une caractéristique de la présente invention, la différence entre les mesures P1 et P2 (ΔPI-P2) provoquant la modification des doses du produit précipitant le phosphore soluble par voie physico-chimique, ou la modification du débit de recirculation des boues est comprise entre 0,1 et 2 mg - P-PO₄ /l, de préférence entre 0,1 et 0,5 mg - P-PO₄ /l.

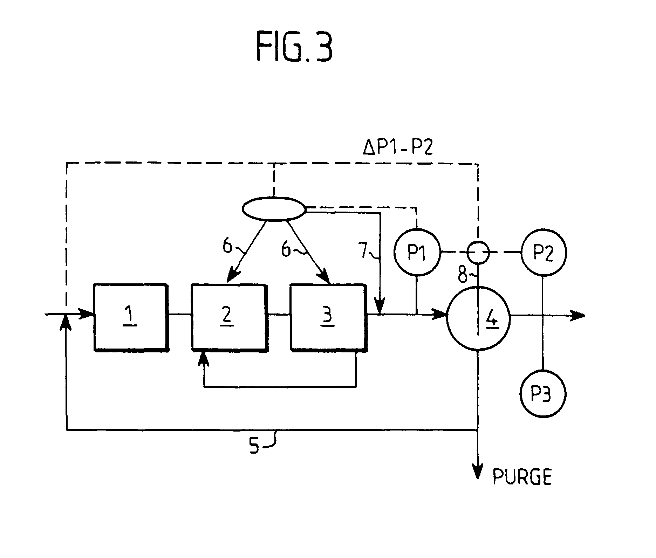

Afin de mieux faire comprendre le procédé objet de la présente invention, on en a décrit ci-après un mode de mise en oeuvre. Il demeure bien entendu qu'il ne s'agit là que d'un exemple n'ayant aucun caractère limitatif. Au cours de cette description,on se réfère à la figure 3 des dessins annexés qui est un schéma illustrant les différentes étapes du procédé selon l'invention.

Sur cette figure 3, on retrouve les zones anaérobie 1, anoxique 2 et aérobie 3 ainsi que le clarificateur secondaire 4 à partir duquel une partie des boues 5 est recyclée vers l'étape anaérobie 1. Le clarificateur secondaire comporte comme connu une purge de boue.

Ainsi qu'on le comprend, le procédé objet de la présente invention comporte pour l'essentiel une mesure de la concentration en phosphore soluble (ortho ou polyphosphates) sur deux endroits de la ligne de traitement, à savoir en amont et en aval du clarificateur secondaire (Figure 3).

Sur la figure 3, la première mesure en continu P1 détermine le niveau du phosphore soluble dans la liqueur sortant de la zone aérobie 3 et gère les injections du produit précipitant 6 dans l'une des zones précédentes 2,3 en fonction des besoins. Cette mesure P1 permet d'ajuster, en fonction de la valeur mesurée, la dose du produit précipitant. La deuxième mesure en continu P2 détermine le niveau du phosphore soluble à la sortie du clarificateur secondaire 4, correspondant au niveau de rejet, et cette mesure est comparée au niveau détecté par la mesure P1, de manière à obtenir une mesure de la différence (ΔPI-P2). L'objectif de la mesure P2 est avant tout de détecter toute anomalie conduisant vers le relargage du phosphore dans le clarificateur secondaire 4.

Selon la présente invention, lorsque la différence ΔPI-P2 entre les deux mesures P1 et P2 dépasse une valeur prédéterminée, fixée généralement entre 0,1 et 2 mg - P-PO₄ /l, et de préférence entre 0,1 et 0,5 mg - P-PO₄ /l, deux types d'actions peuvent être déclenchées :
- Dans un premier temps, la dose du produit précipitant, initialement déterminé par la mesure P1 peut être complétée par une seconde injection 7 de ce produit précipitant, effectuée avant la zone de clarification secondaire 4, afin que la précipitation du phosphore relargué dans le clarificateur puisse avoir lieu grâce à l'excès du produit précipitant injecté. Cette injection secondaire peut être mise en oeuvre de façon continue ou discontinue dans l'une des zones précédant la clarification secondaire 4, ou à l'entrée de la zone de clarification secondaire. Elle peut être également effectuée au même endroit que la première injection gérée par la mesure P1.
- Une deuxième action consiste à agir directement au niveau des boues relarguant le phosphore, en réduisant leur temps de séjour dans le clarificateur secondaire 4. Ceci est effectué grâce à une augmentation du débit 5 de recirculation des boues . Cette action peut être couplée à une mesure du niveau de voile des boues 8.

Toujours selon le schéma de la figure 3, le niveau de sortie de l'eau traitée est également contrôlé par une mesure P3 de la turbidité, afin de permettre de détecter les éventuelles pertes en MeS, qui entraîneraient une augmentation de la concentration en phosphore particulaire du rejet.

Selon la présente invention, les mesures de la concentration en pnospnore soluble de l'eau traitée, P1 et P2 peuvent être obtenues soit à partir de deux appareils de mesure différents, soit à partir d'un appareil unique à deux prises.

On retiendra de la description qui précède que le procédé objet de la présente invention se distingue nettement de l'état antérieur de la technique rappelé ci-dessus (DE-39 02 626) notamment en ce qu'il porte sur un traitement simultané par voie biologique et physico-chimique formellement déconseillé dans cet état antérieur de l'art. Par ailleurs, dans l'invention, et contrairement à l'état antérieur de la technique, on n'avise pas à améliorer la dénitrification, par conséquent, on ajoute pas de carbone organique dans l'effluent à traiter.

Les avantages du procédé objet de la présente invention, par rapport à l'état antérieur de la technique de déphosphatation, sont notamment les suivants :
- détection en continu de toute anomalie ou de tout défaut au niveau de relargage du phosphore dans le clarificateur,
- contrôle et meilleure fiabilité du niveau de rejet en phosphore.
- meilleure asservissement des doses du produit précipitant, ce qui se traduit par des économies,
- optimisation de la combinaison des déphosphatations biologique et physico-chimique.

Il demeure bien entendu que la présente invention n'est pas limitée aux exemples de mises en oeuvre décrits et/ou représentés mais qu'elle en englobe toutes les variantes.

## Revendications

1. Procédé de traitement des eaux usées par voie biologique et physico-chimique, mettant en oeuvre une déphosphatation biologique, ce procédé comportant des étapes de traitements anaérobie, anoxique et aérobie, suivies d'une étape de clarification secondaire, l'eau usée étant mélangée à la boue biologique recyclée à partir de l'étape de clarification secondaire dans la première étape anaérobie, afin d'effectuer un relargage du phosphore dans la phase soluble par les bactéries poly-P, ledit mélange subissant ensuite les étapes traditionnelles de traitements anoxique et aérobie, la boue activée étant séparée de l'eau traitée dans l'étape de clarification secondaire et elle est ensuite partiellement recyclée dans l'étape anaérobie, en tête de la ligne de traitement, et partiellement extraite du système, ce procédé étant caractérisé en ce que :
- la concentration en phosphore soluble de l'eau traitée est mesurée sur au- moins deux endroits différents de la ligne de traitement : à l'entrée de l'étape de clarification secondaire afin d'obtenir une mesure P1 et à la sortie de la ligne de traitement pour obtenir une mesure P2, la mesure P1 déterminant les doses du produit précipitant le phosphore soluble par voie physico-chimique dans la liqueur d'au moins l'un des bassins ou s'effectuent les étapes anoxique ou aérobie en amont de la clarification secondaire ;
- la mesure P2 est comparée en permanence à la mesure P1 afin de détecter un éventuel relargage du phosphore au niveau de la clarification secondaire et,
- lorsque la différence des deux mesures (ΔP1-P2) est constatée, on déclenche au moins l'une des deux mesures suivantes :
- on réalise une injection supplémentaire du produit précipitant, avant la clarification secondaire, afin que la précipitation du phosphore relargué au cours de l'étape de clarification secondaire puisse avoir lieu grâce à l'excès du produit précipitant injecté;
- on modifie le débit de recirculation des boues de l'étape de clarification vers la première étape anaérobie, afin de réduire leur temps de séjour durant cette étape de clarification

2. Procédé selon la revendication 1, caractérisé en ce que les injections du produit précipitant le phosphore soluble par voie physico-chimique sont mises en oeuvre de façon continue au cours de l'une des étapes précédant la clarification.

3. Procédé selon la revendication 1, caractérisé en ce que les injections du produit précipitant le phosphore soluble par voie physico-chimique sont mises en oeuvre de façon discontinue au cours de l'une des étapes précédant la clarification.

4. Procédé selon l'une quelconque des revendications précédentes, caractérisé en ce qu'il comporte une mesure de la turbidité de l'effluent final, mise en oeuvre de façon continue.

5. Procédé selon l'une quelconque des revendications précédentes, caractérisé en ce que l'injection supplémentaire du produit précipitant le phosphore en phase soluble par voie physico-chimique est effectuée au même endroit que l'injection gérée par la mesure P1.

6. Procédé selon l'une quelconque des revendications 1 à 4, caractérisé en ce que l'injection supplémentaire du produit précipitant le phosphore en phase soluble par voie physico-chimique est effectuée à l'entrée de l'étape de clarification.

7. Procédé selon l'une quelconque des revendications précédentes, caractérisé en ce que la différence entre les mesures P1 et P2 (ΔP1-P2) provoquant la modification des doses du produit précipitant le phosphore soluble par voie physico-chimique ou la modification du débit de recirculation des boues se situe dans un intervalle de 0,1 à 2 mg P-PO₄/l, de préférence 0.1-0.5 mg P-PO₄/l.

8. Procédé selon l'une quelconque des revendications précédentes, caractérisé en ce que les mesures de la concentration du phosphore soluble P1 et P2 sont assurées par deux appareils différents.

9. Procédé selon l'une quelconque des revendications 1 à 7, caractérisé en ce que les mesures de la concentration du phosphore soluble P1 et P2 sont assurées par un appareil à deux prises.

## Patentansprüche

1. Verfahren zur Behandlung von Abwasser auf biologischem und physikochemischem Wege, indem eine biologische Dephosphatierung durchgeführt wird, wobei das Verfahren die Stufen einer anaeroben, anoxischen und aeroben Behandlung mit anschließender Nachklärstufe umfaßt und das Abwasser mit Belebtschlamm vermischt wird, der von der Nachklärstufe in die anaerobe erste Stufe rezirkuliert wird, um ein Aussalzen des Phosphors in der löslichen Phase durch Poly-P-Bakterien stattfinden zu lassen, wonach das Gemisch den herkömmlichen Stufen einer anoxischen und einer aeroben Behandlung unterworfen wird, wobei der Belebtschlamm vom behandelten Wasser in der Nachklärstufe abgetrennt und anschließend teilweise in die anaerobe Stufe am Anfang der Behandlungslinie rezirkuliert und teilweise dem System entzogen wird, und dieses Verfahren **dadurch gekennzeichnet ist, daß**
- die Konzentration des löslichen Phosphors im behandelten Wasser an mindestens zwei verschiedenen Stellen der Behandlungslinie: am Eingang der Nachklärstufe, um einen Meßwert P1 zu erhalten, und am Ausgang der Behandlungslinie, um einen Meßwert P2 zu erhalten, gemessen wird, wobei der Meßwert P1 die Dosis des Mittels zur Ausfällung des löslichen Phosphors auf physikochemischem Wege aus der Flüssigkeit wenigstens eines der Becken bestimmt, worin vor der Nachklärstufe die anoxische oder die aerobe Stufe stattfindet,
- der Meßwert P2 ständig mit dem Meßwert P1 verglichen wird, um gegebenenfalls ein Aussalzen des Phosphors in der Nachklärstufe nachzuweisen, und,
- wenn die Differenz der beiden Meßwerte (ΔP1-P2) festgestellt ist, wenigstens eine der zwei folgenden Maßnahmen ausgelöst wird:
- vor der Nachklärstufe wird zusätzliches Fällungsmittel zugeführt, damit in dieser auf Grund des Überschusses an zugegebenem Fällungsmittel die Ausfällung des ausgesalzten Phosphors stattfinden kann, und
- der Volumenstrom des Rückflusses des Schlamms von der Nachklärstufe zur anaeroben ersten Stufe wird verändert, um dessen Verweilzeit in der Nachklärstufe zu verkürzen.

2. Verfahren nach Anspruch 1, **dadurch gekennzeichnet, daß** die Zufuhr des Mittels, das den löslichen Phosphor auf physikochemischem Wege ausfällt, in einer der Stufen, die der Klärung vorhergehen, kontinuierlich erfolgt.

3. Verfahren nach Anspruch 1, **dadurch gekennzeichnet, daß** die Zufuhr des Mittels, das den löslichen Phosphor auf physikochemischem Wege ausfällt, in einer der Stufen, die der Klärung vorhergehen, diskontinuierlich erfolgt.

4. Verfahren nach einem der vorhergehenden Ansprüche, **dadurch gekennzeichnet, daß** es eine kontinuierliche Trübungsmessung des abgegebenen Abprodukts umfaßt.

5. Verfahren nach einem der vorhergehenden Ansprüche, **dadurch gekennzeichnet, daß** die zusätzliche Zufuhr des Mittels, das den Phosphor auf physikochemischem Wege aus der löslichen Phase ausfällt, an derselben Stelle wie die vom Meßwert P1 bestimmte Zufuhr erfolgt.

6. Verfahren nach einem der Ansprüche 1 bis 4 , **dadurch gekennzeichnet, daß** die zusätzliche Zufuhr des Mittels, das den Phosphor auf physikochemischem Wege aus der löslichen Phase ausfällt, am Eingang der Klärstufe erfolgt.

7. Verfahren nach einem der vorhergehenden Ansprüche, **dadurch gekennzeichnet, daß** die Differenz zwischen den Meßwerten P1 und P2 (ΔP1-P2), welche die Veränderung der Dosis des Mittels, das den löslichen Phosphor auf physikochemischem Wege ausfällt, oder die Veränderung des Volumenstroms des Rückflusses des Schlammes bewirkt, innerhalb eines Intervalls von 0,1 bis 2 mg P-PO₄/l und vorzugsweise 0,1 bis 0,5 mg P-PO₄/l liegt.

8. Verfahren nach einem der vorhergehenden Ansprüche, **dadurch gekennzeichnet, daß** die Messungen der Konzentration des löslichen Phosphors P1 und P2 von zwei verschiedenen Geräten durchgeführt werden.

9. Verfahren nach einem der Ansprüche 1 bis 7, **dadurch gekennzeichnet, daß** die Messungen der Konzentration des löslichen Phosphors P1 und P2 von einem Gerät mit zwei Meßstellen durchgeführt werden.

## Claims

1. Process for the biological and physicochemical treatment of effluents, using a biological dephosphatization, said process including anaerobic, anoxic and aerobic treatment stages, followed by a secondary clarification stage, the effluent being mixed with biological sludge recycled from the secondary clarification stage into the primary anaerobic stage, in order to effect a salting out of the phosphorus in the soluble phase by poly-P bacteria, said mixture then undergoing conventional anoxic and aerobic treatment stages, the activated sludge being separated from the effluent in the secondary clarification stage and is then partly recycled into the anaerobic stage, at the top of the treatment line and partly extracted from the system, said process being characterized in that:
- the soluble phosphorus concentration of the treated water is measured at at least two different locations of the treatment line, namely at the start of the secondary clarification stage in order to obtain a measurement P1 and at the outlet of the treatment line for obtaining a measurement P2, the measurement P1 determining the doses of the product physicochemically precipitating the soluble phosphorus in the liquor of at least one of the tanks where the anoxic or aerobic stages take place upstream of the secondary clarification,
- measurement P2 is permanently compared with measurement P1 in order to detect a possible salting out of phosphorus at the secondary clarification stage and
- when the difference between the two measurements (ΔP1-P2) is noted, at least one of the two following measures is initiated:
- there is a supplementary injection of the precipitating product, prior to the secondary clarification, so that the precipitation of the phosphorus salted out during the secondary clarification stage can take place as a result of the excess of the injected precipitating product,
- the recirculation flow rate of the sludge of the clarification stage to the primary anaerobic stage is modified, in order to reduce the residence time thereof during said clarification stage.

2. Process according to claim 1, characterized in that the injection of the product physicochemically precipitating the soluble phosphorus are performed continuously during one of the stages preceding clarification.

3. Process according to claim 1, characterized in that the injections of the product physicochemically precipitating the soluble phosphorus are performed discontinuously during one of the stages preceding clarification.

4. Process according to any one of the preceding claims, characterized in that it includes a measurement of the turbidity of the final effluent, which is carried out continuously.

5. Process according to any one of the preceding claims, characterized in that the supplementary injection of the product physicochemically precipitating the phosphorus in the soluble phase takes place at the same location as the injection controlled by the measurement P1.

6. Process according to any one of the claims 1 to 4, characterized in that the supplementary injection of the product physicochemically precipitating the phosphorus in the soluble phase takes place at the intake of the clarification stage.

7. Process according to any one of the preceding claims, characterized in that the difference between the measurements P1 and P2 (ΔP1-P2) giving rise to the modification of the doses of the product physicochemically precipitating the soluble phosphorus or the modification of the sludge recirculation flow rate is in a range 0.1 to 2 and preferably 0.1 to 0.5 mg P-PO₄/I.

8. Process according to any one of the preceding claims, characterized in that the concentration measurements of the soluble phosphorus P1 and P2 are carried out by two different apparatuses.

9. Process according to any one of the claims 1 to 7, characterized in that the concentration measurements of the soluble phosphorus P1 and P2 are performed by one apparatus having two intakes.
